# EUROPEAN PATENT APPLICATION

(11) **EP 1 070 504 A1**
(43) Date of publication of application: **24.01.2001**
(21) Application number: 00902102.3
(22) Date of filing: 04.02.2000
(51) Int. Cl.: A61K 35/78, A61K 35/74, A61K 38/00, A61P 9/10, A61P 37/04, A23L 1/20, A23L 1/30, A23L 1/31, A23L 1/32, A23K 1/16

(54) **MATERIALS FOR PREVENTING ARTERIOSCLEROSIS, IMMUNOPOTENTIATING MATERIALS, VERTEBRATES FED WITH THESE MATERIALS AND EGGS THEREOF**

(30) Priority: 04.02.1999 JP 2749699
(71) Applicant: Nichimo Co., Ltd, Tokyo 140-0002 (JP)
(72) Inventor: TAKEBE, Minoru, Shinagawa-ku, Tokyo 140-0002 (JP)
(74) Representative: Ouzman, Beverley Nicola Claire
(86) International application number: JP0000627
(87) International publication number: WO0045830

(57) **Abstract**

An arteriosclerosis-preventing material, immune-activating material, vertebrate that has eaten these, and eggs of such vertebrate. Such materials are produced by inoculating koji mold on a pulse crop to effect koji preparation and adding water to a product of the koji preparation, thus hydrolyzing proteins and/or saccharides contained in the product of said koji preparation and by utilizing the activity common to lactic acid bacteria naturally contained in the starting materials and lactic acid bacteria that is added to the product of the koji preparation. An arteriosclerosis-preventing material possessing arteriosclerosis-preventing capability, an immune-activating material possessing immune-activating capability that are effective in maintaining the health of vertebrates, a vertebrate other than a human being that has been reared by adding these arteriosclerosis-preventing material and immune-activating material to feed, and the egg of such a vertebrate, are obtained. The necessary amount of arteriosclerosis-preventing material and immune-activating material that are the product of the present invention is added to, for instance, food products, fodder, feed, etc. and absorbed *in vivo* from the digestive tract of vertebrates to realize arteriosclerosis-preventing capability and immune-activating capability, thus safely preventing the onset of arteriosclerosis-related disease in such a vertebrate as well as improve immunity of the vertebrate, thus effectively accomplishing a maintenance and promotion of good health and a prevention of disease, etc.

## Description

### Technical Field of the Invention

The present invention relates to an arteriosclerosis-preventing material and an immune-activating material, the starting material of which being pulse crops, and a vertebrate that have eaten these, as well as eggs of such vertebrate; and more particularly to an arteriosclerosis-preventing material possessing arteriosclerosis-preventing capability that prevents arteriosclerosis of circulatory system and related diseases caused thereby in vertebrates, including human beings, animal fowl, and fish (referred to hereafter as "vertebrates"), to an immune-activating material possessing immune-activating capability that improves the immune capability of vertebrates, to a vertebrate other than a human being that has been reared eating feed to which the arteriosclerosis-preventing material and immune-activating material are partially added, and to eggs of such vertebrate.

The term pulse crop in the present invention refers to leguminous corps, such as soybeans, etc., and their leguminous crops, processed products, etc.

### Background Art

In general, there is a strong demand for products of which starting material is pulse crop, and many types of products are being presented.

Soy sauce, miso, etc. are conventional products of which starting material is pulse crop; and such products are fermentation products that are made using koji mold. Koji mold is mainly used for saccharification of starch and for decomposition of proteins in these conventional fermentation products. Moreover, it is also known that a manufacture of products from these fermentation products with koji mold only is difficult and therefore the products of these are manufactured by utilizing the activity of lactic acid bacteria and yeast, etc. This means that the properties of the koji mold are employed.

Nevertheless, almost all of these products are specialty food products, and it is no exaggeration to say that there are no conventional food products that can be eaten in large quantities for the purpose of nutrition and maintaining health.

Therefore, the inventor of the present patent application has presented food products, fodder, feed, etc., that can be eaten in large quantities with a high isoflavone aglycone content and are made by koji preparation performed on pulse crop, such as soybeans, etc., as the starting material using koji mold and hydrolyzing this product to completely decompose and eliminate the phytic acid (see Japanese Patent Application Laid-Open (Kokai) No. H7-23725, International Publication No. WO 95/16362, etc.).

The inventor completed the present invention based upon a discovery that products, of which starting material being the pulse crops that are vegetables, can very effectively prevent disease such as arteriosclerosis, etc. which derives from today's change in eating habits, particularly an increase in consumption of fat, such as cholesterol, etc.

Modern man has been released from the poverty that persisted for many years and entered an era in which it can be said that advances in medicine have resulted in prolonged life. There is also the gluttony of developed countries. Therefore, the risk of chronic diseases that can also be called a diseases associated with lifestyle has increased as a result of diet and stress. The inventor has found that onset of arteriosclerosis and reduced immunity are very closely related and hoped to develop food products that are capable of preventing arteriosclerosis using the same component as estrogen without reducing immunity, even under stress. It is a known fact that the isoflavone contained in pulse crops, such as soybeans, etc. has weak estrogen-like activity. However, there are no reports of study of the effect of prevention and treatment of arteriosclerosis with food products, fodder, feed, etc., with a high isoflavone aglycone content. On the other hand, there is also no report of immune-activating capability of products that are obtained by fermentation of koji mold using pulse crops, such as soybeans, etc. The inventor completed the present invention upon discovering that the product obtained by the same production method has a arteriosclerosis-preventing capability and immune-activating capability.

More specifically, in the perimenopausal female at age 45-55, total cholesterol (hereafter referred to as TC) increases; and lipometabolism anomalies develop, such as a gradual reduction in high-density lipoprotein cholesterol (hereafter referred to as HDL-C) and an increase in low-density lipoprotein cholesterol (hereafter referred to LDL-C), etc. The risk of death due to ischemic heart disease thus becomes higher than that of men of same age group. The reason for this is a reduction in the secretion of the female hormone (estrogen) after menopause. Consequently, an attempt is made to prevent arteriosclerosis by estrogen replacement therapy, etc. However, there is the risk of adverse effects, such as uterine cancer, etc., due to excess hormone activity when synthetic estrogen preparations are used.

The mechanism of estrogen activity is can be described as below. It has been made clear that estrogen inhibits synthesis and secretion of cytokine interleukin 1 (IL-1) interleukin 6 (IL-6) and tumor necrosis factor (TNF), and promotes synthesis of TGF-β (transforming growth factor) (Eriksen, E. F. et al.: Science, 241:84-85, 1988). It also appears that excess production of TNF and IL-1 induced by a reduction in estrogen secretion is connected with lipometabolism anomalies. Moreover, such cytokines are also related to changes in immune capability induced by stress. Thus, there is a need for development of food products that are very effective in terms of both arteriosclerosis-preventing capability and immune-activating capability.

Arteriosclerosis will be explained below in further detail. There is a dramatic increase in serum cholesterol levels, particularly due to an increase in consumption of animal fats, when meat is added to the diet; and arteriosclerosis derived therefrom becomes a problem. Consequently, the first countermeasure for preventing the arteriosclerosis is to reduce total serum cholesterol levels; and further, reducing the HDL-C, LDL-C and very-low-density lipoprotein cholesterol (hereafter referred to as VHDL-C), particularly serum LDL-C, which comprise serum cholesterol, is also necessary. This is because oxidized LDL-C is a factor that promotes arteriosclerosis, and it facilitates the development of disease such as arteriosclerotic heart disease, etc. This is supported by international epidemiological study reports which indicate that the amount of cholesterol consumed and the mortality rate due to arteriosclerotic heart disease are closely related, and that when compared to East Asians, Europeans and Americans have a very high ischemic heart disease-induced mortality rate due to the amount of cholesterol consumed. Nevertheless, it has also recently been reported that serum cholesterol levels in Japan, particularly among the younger generation, are higher than those of Americans of the same age in studies of total cholesterol levels. Therefore, it is estimated that this problem will become serious in Japan in the future.

Consequently, once arteriosclerosis has developed, there is no effective therapeutic method; and it eventually becomes a lethal symptom referred to as a thrombus. It is reported that the risk of this thrombus is higher in middle age than when younger. Hemangioendothelial cells not only present as physical barriers between blood components and tissue but also produce and secrete many bioactive substances in addition to antithrombosis, selective permeability of substances, promotion of coagulation and fat metabolism, thus positively participating in maintenance of body functions.

Acetyl choline and prostacycline are vasorelaxing substances that affect this hemangioendothelium, and there are reports that prostacycline is reduced by as much as 1/3 - 1/4 that of young people in middle-aged and elderly people (Yanagisawa, M., et al. (1988) 332, 411-415). It is also reported that the amount of endothelin (ET), which is a vasoconstrictor, is more than twice among older age groups when compared to younger age groups (Tokunaga, O., et al.: Lab Invest (1992) 67, 210-217). LDL-C is taken up intracellularly via LDL-C receptors of hemangioendothelial cells. Uptake of oxidized LDL-C is high, and this plays an important role in advancing the development of arteriosclerosis. It is reported that this uptake is higher among hemangioendothelial cells of older people than younger people (Hashimoto, M., et al.: Exp Gerontology (1991) 26, 397-406). Thus, changes in morphology and capability of hemangioendotheial cells develop during a person's lifetime, and further intense deterioration at sites of morbid change, such as arteriosclerosis, etc. becomes a problem with old age. Preventing changes on the hemangioendothelial cell level is extremely important as a countermeasure to disease caused by arteriosclerosis.

Accordingly, there is a demand for development of an arteriosclerosis-preventing material that has excellent activity as a material which prevents arteriosclerosis (for instance, prophylactic food) in both the young and the old.

On the other hand, regardless of the fact that the nutritional state in the developed countries is good, immunity to infection has not been improved; and food allergies and hay fever have newly become serious problems. Moreover, animals are raised under dense grazing condition from the time they are very young in order to make economic stock breeding productive. This results in stress and a reduction in immunity, and thus far there has been no reduction in the mortality rate due to infection; and antibiotics are frequently administered.

Moreover, immune-activating capability is very important in maintaining health among vertebrates, and antibacterial agents have been developed for this purpose. Asano et al. (Asano et al., *Chikusan no Kenkyu*, 48(11): 63-65(1994)) report that when they investigated the effect of administration of preparations of live spores and the cell wall of *Clostridium butyricum,* an obligate anaerobic bacterium, on immune response, there was some type of immunopotentiating effect. Tanaka et al. reports that when they administered this bacterium to germ-free quails (Tanaka et al., "Mukin Seibutsu" (J. germfree life gnotobil.), vol. 21, No.2,80-82(1991)), anomalies accompanied by emphysema and inflammation of the caecum were observed to with feed to which lactose had been added. Furthermore, this *Clostridium butyricum* is an intestinal flora, but there have also been reports of newborn deaths associated with enteritis and therefore, this is a problem point.

Consequently, it can be said that obtaining immune-activating activity with lactic acid bacteria that do not have this type of detrimental effect on vertebrates would be safer and more effective.

Therefore, immunopotentiation that uses lactic acid bacteria (genus *Enterococcus*), which is one type of human intestinal flora, has been presented in Japanese Patent Application Laid-Open (Kokai) Nos. 7-238024 and 8-99887.

Nevertheless, the immune-activating materials described in these publications are cultivation products of lactic acid bacteria only and therefore use only the properties of the lactic acid bacteria.

Meanwhile, the immune system can be divided into two: a general immune system and a local immune system; and attention is being focused on the intestinal mucosa immune system as a local immune system to oral infection. In this immune system, it is necessary to increase IgA antibody, and discovering of an antigen that effectively increases IgA antibody is important There are reports that it is possible to increase IgA antibody and reduce IgE antibody of the general immune system by way of inducing immune tolerance with effective antigen that is not toxic. This can be expected to have a connection with reduction of IgE antibody, which is the source of food allergies and hay fever, by enhancing the intestinal mucosa immune system.

### Disclosure of the Invention

The present invention is based on the above-described points. The object of the present invention is to provide: products which are obtained by using the activity realized by coexistence with lactic acid bacteria after cultivation and propagation of koji mold in pulse crops as the starting material, the products being an arteriosclerosis-preventing material that has an arteriosclerosis-preventing capability and an immune-activating material that has an immune-activating capability which are effective in maintaining the health of vertebrates; and to provide a vertebrate other than a human being that has been reared with these arteriosclerosis-preventing material and immune-activating material added to feed; and also to provide eggs of such a vertebrate.

Another object of the present invention is to provide an arteriosclerosis-preventing material and an immune-activating material whose arteriosclerosis-preventing capability and immune-activating capability are realized by addition thereof in the necessary amount to food products, fodder, feed, etc.

The inventor of the present application performed intense research in order to accomplish the above-described objects and, as a result, completed the present invention. The invention is based upon the discovery of a product that possesses arteriosclerosis-preventing capability, along with cholesterol level-reducing capability, and immune-activating capability, which are excellent capabilities for promoting health of vertebrates; and such a product is produced by inoculating koji mold on a pulse crop to effect koji preparation and then hydrolyzing the proteins and/or saccharides contained in the product of koji preparation by adding water to this product of the koji preparation, or such a product is produced as described above and further by promoting propagation of lactic acid bacteria contained in the product of the koji preparation and/or of lactic acid bacteria that has been added to the product of the koji preparation during the hydrolysis.

The reason how the present invention was completed is explained below in further detail. Arteriosclerosis-preventing capability can be expected from the above-described products because first the products have the effect of reducing the total amount of serum cholesterol of vertebrates, secondly the products have the effect of preventing oxidation of LDL-C which has a detrimental effect on arteriosclerosis, and third the products have the effect of macrophage decomposition of cholesterol esters in foam cells that are generated with the uptake of oxidized LDL-C. Furthermore, arteriosclerosis-preventing capability can be expected because the product produced as described above has a vasorelaxing effect. Immune-activating capability can further be expected because the product produced as described above has immune-activating capability.

These effects will now be described in further detail.

The Food and Drug Administration (FDA) of the United States has recognized the fact that soybean protein, which contains isoflavone, has the ability to prevent an increase in serum cholesterol levels and has said that it is performing studies in order to promote to prevention of heart disease among Americans based on the health claim of consuming soybean proteins. On the other hand, Sugano et al. (Sugano, M., Goto, S., Yamada, Y., Yoshida, K., Hashimoto, Y., Matsuo, T., and Kimoto, M. (1990), J. Nutr., 120: 977-985.) report that because intestinal absorption of bile acids is obstructed by the intervention of soybean protein undecomposed product or non-absorbing peptides, the high-molecular-weight fraction (referred to hereafter as HMF) that remained after treatment of isolated soybean protein with protease derived from microorganisms shows better ability to bind with bile acids and to reduce total serum cholesterol levels than isolated soybean protein itself. Moreover, Anthony et al. (Anthony, M., Clarkson, T. B, Hugbes, C. L., Jr., Morgan, T. M. and Burke, G. L., (1996) J. Nutr, 126:43-50) report on the prophylactic effect of soybean isoflavone on hypercholesterolemia in monkeys. Thus, it is clear that the above-described products of the present invention have the ability to reduce total serum cholesterol. On the other hand, there are reports that state that cultivated lactic acid bacteria has a reducing effect on cholesterol and neutral fats (Japanese Patent Application Publication (Kokoku)Nos. 4-73984, 5-26768, 142280 and 3-65362). These have shown that the cellular wall components of lactic acid bacteria are active in terms of reducing cholesterol and neutral fats.

The arteriosclerosis-preventing capability of antioxidants will be further described below. When the product with arteriosclerosis-preventing capability of the present invention is ingested, it moves through the bloodstream, where the components with antioxidant effects of the product have been absorbed, so as to have on inhibiting effect of oxidation of lipid components in the blood, particularly LDL-C. Therefore, effective prevention of arteriosclerosis can be expected. This antioxidant effect can be evaluated based on the ability to eliminate active oxygen elimination capability and obstruction of lipoxygenase.

Polyphenolic antioxidants are generally contained in vegetable food products. For instance, flavonoids are contained in tea, red wine, onions, apples, etc. A report says that this flavonoid component inhibits oxidation of blood LDL-C (refer to Ishikawa, T., Suzukawa, M., Ito, T., Yoshida, H., Ayaori, M., Nishiwaki, M., Yonemura, A., Hara, Y., and Nakamura, H.: Am. J. Clin. Nutr., 66, 261 (1997)). Luiz da Silva reports that activity of 15-lipoxygenase, which is an enzyme that participates in the oxidation of LDL-C, is obstructed by quercetin, which is a flavonoid contained in onions, thus indicating that flavonoid component are effective in inhibition of LDL-C oxidation.

Nevertheless, there are no reports on the blood LDL-C oxidation-inhibiting effect of isoflavonoids contained in pulse crops, particularly soybeans. However, the inventor focused on the fact that soybean fermentation products previously presented by the inventor can inhibit oxidation of blood LDL-C.

More specifically, Cai et al, (Cai, Q., Wei, H.,: Nutr. Cancer (1996) 25,1 - 7) report that when genistein was added to feed and administered to SENCAR mice, catalase was introduced in the small intestine, SOD (superoxide dismutase) was introduced in the skin, and glutathione-S-transferase activity was increased in the skin, small intestine, liver, kidneys and lungs. This is said to be the effect of isoflavone aglycone. Consequently, because the product of the present invention contains isoflavone aglycone, the results described by the authors can be expected in full.

The inventor confirms that SOD is produced by the product of the present invention. In other words, SOD-like activity of fermented soybean embryo arid fermented defatted soybean produced by the method of the present invention using *Aspergillus oryzae* as koji mold was compared to two types of starting materials, soybean embryo (cooked) and defatted soybean (cooked). The result was that no SOD-like activity was detected from two types of starting materials, either soybean embryo (cooked) or defatted soybean (cooked); to the contrary, the fermented soybean embryo and the fermented defatted soybean of the present invention respectively show excellent SOD-like activity at 570 units/g and excellent SOD-like activity at 370 units/g (detection limit of 30 units/g).

Oxidation of LDL-C in the blood occurs as a result of superoxides reacting with LDL-C. SOD has the effect of decomposing active oxygen. Therefore, prevention of LDL-C oxidation can be expected by ingesting the fermented soybean product of the present invention that has high SOD-like activity. Oxidation of LDL-C leads to arteriosclerosis. Thus, results in terms of reliable prevention of arteriosclerosis can be expected since oxidation of LDL-C is prevented to the utmost.

Furthermore, it appears that when oxidized LDL-C is taken up by macrophages, the ability of such isoflavone aglycone to inhibit arteriosclerotic disease is realized. In other words, when the neutral cholesterol esterase (N-CEase) of macrophages is activated, the cholesterol in foam cells that have accumulated is decomposed and eliminated. Thus, this N-CEase activity prevents arteriosclerosis. For instance, Tomita et al. (Tomita, T. et al. (1996) Biochim. Biophys. Acta, 1300, 210 - 218) confirm, on the cellular level, that estradiol (17β-estradiol (E2)), which is a female hormone, induces N-CEase activity. Consequently, isoflavone aglycone has female hormone-like activity, and the same activity as E2 in terms of binding with receptors to prevent arteriosclerotic disease can be expected.

When macrophages are discussed, it appears that the cytokine itself that is released by macrophages has an effect on vasorelaxation ability. More specifically, interleukins (hereafter referred to as IL), which are cytokines as active factor, such as IL-6, also have the ability to promote thrombogenesis. Activation of this type of IL is even more of a problem, and IL-1 and IL-2 are shown to increase production of prostacycline (PG12), which has a vasorelaxing effect This indicates that although the speed-regulating enzyme in arachidonic acid metabolism is cycloxygenase (referred to hereafter as COX), IL-1 induces the manifestation of COX, and IL-2 also induces the manifestation of COX (see Maruo et al. "Igaku no Ayumi," (1994) vol. 170, No.6,426-429). Consequently, the cytokine produced by the product of the present invention can be expected to have an effect on cellular immunity of helper T cell type 1 (Th1) and thus produces IL-2, induces the production of prostacycline and relaxes blood vessels. Consequently, a prophylactic effect on thrombogenesis induced by arteriosclerosis can be expected.

Furthermore, the inventor and others confirm that, when compared to untreated defatted soybeans, the product of the present invention induces enzyme activity of cytochrome P-450 in the liver of mice. Toda et al. (Toda, Noboru, Okamura, Tomb: Nihon Yakuri Zasshi (Soutou) (1990) 95, 295-376) report that it is possible that cytochrome P-450, which is known as a metabolite of arachidonic acid, participates in the production of endothelium-derived relaxing factor: (EDRF). Consequently, the product of the present invention can be expected to have a vasorelaxing effect induced by said EDRF and the ability to inhibit to platelet aggregation, as well as anti-thrombotic activity on nodular arteriosclerosis by way of these effects.

As described above, the product having arteriosclerosis-preventing capability of the present invention is characterized in that prevention of a variety of diseases caused by arteriosclerosis can be expected.

As previously described, soybean protein has the ability to reliably obstruct absorption of bile acids from the intestine, and therefore, effects in terms of reducing blood cholesterol levels can be expected. However, it is necessary to consume large amounts of soybean proteins in order to realize this effect. Moreover, flavonoids have the ability to reliably inhibited oxidation of LDL-C; but because they do not have estrogen activity, the only result that is expected is inhibition of oxidation, and the arteriosclerosis-preventing effect is very weak. The arteriosclerosis-preventing material that is the product of the present invention is mainly fermented product containing isoflavone aglycone, etc. produced by fermentation with koji mold. In particular, the product of the present invention, which is mainly the isoflavone aglycone daidzein, has weak estrogen-like effects, and it has been confirmed that it has the capability of binding with estrogen receptor and accelerating activity a of LDL-C receptor in the liver. This arteriosclerosis-preventing capability is not seen with soybean proteins and flavonoids. Moreover, it is confirmed that isoflavone aglycone and fermented product have strong antioxidant capability; and therefore, sufficient results in terms of arteriosclerosis-preventing activity can be expected. Estrogen effects, including prevention of hyperlipidemia, which is a cause of arteriosclerosis, prevention of oxidation of LDL-C, improved sugar metabolism, vasodilation of vascular walls, inhibition of propagation of tunica media smooth muscle cells, etc. can be all expected.

Moreover, the product of the present invention is obtained by koji preparation and hydrolysis of pulse crops as the starting material while simultaneously propagating lactic acid bacteria in large quantities. Immune-activating capability by the large amounts of lactic acid bacteria that have propagated can be expected. Extremely excellent immune-activating capability can be expected because the isoflavone aglycone that has been produced by the product of the present invention is easily absorbed by blood vessels and stimulates macrophages to improve immunity.

The structure of the present invention and its effects and results will be further described.

This type of arteriosclerosis-preventing material of the present invention is characterized in that it is a product that has an arteriosclerosis-preventing capability and is produced by inoculating koji mold on a pulse crop to effect koji preparation and adding water to the resulting product of the koji preparation, thus hydrolyzing the proteins and/or saccharides contained in such a resulting product. This arteriosclerosis-inhibiting material has arteriosclerosis-inhibiting capability; and when it is absorbed *in vivo* from the digestive tract of vertebrates, such as humans, livestock, fowl, fish, etc., the onset of arteriosclerosis in the vertebrates can be reliably prevented; and maintenance and promotion of good health and prevention of disease, etc. are well accomplished.

Moreover, another arteriosclerosis-preventing material of the present invention is characterized in that it is a product that has an arteriosclerosis-preventing capability and is produced by inoculating koji mold on a pulse crop to effect koji preparation and by adding water to the resulting product of the koji preparation, thus hydrolyzing the proteins and/or saccharides contained in such a resulting product; and further, upon this hydrolysis, by promoting a propagation of lactic acid bacteria contained in resulting product of the koji preparation and/or the lactic acid bacteria that have been added to the resulting product of the koji preparation. This arteriosclerosis-preventing material possesses arteriosclerosis-preventing capability. Therefore, when it is absorbed *in vivo* from the digestive tract of vertebrates, such as humans, livestock, fowl, fish, etc., arteriosclerosis-preventing capability is realized, and as a result, the onset of arteriosclerosis in the vertebrates is reliably prevented; and maintenance and promotion of good health and prevention of disease, etc., are well accomplished. Furthermore, when lactic acid bacteria that has propagated is absorbed *in vivo* from the digestive tract of the vertebrates, there will be a reduction in the total amount of cholesterol by the lactic acid bacteria; and as a result, arteriosclerosis-preventing capability is enhanced even further.

The above-described lactic acid bacteria is the genus *Enterococcus,* particularly at least one species of *Enterococcus faesium* and *Enterococcus faecalis*. By using this species of lactic acid bacterium, it is possible to realize even better arteriosclerosis-preventing capability.

Moreover, the above-described arteriosclerosis-preventing material is characterized in that the product that has the above-described arteriosclerosis-preventing capability is in concentrated form. Because the product possessing arteriosclerosis-preventing capability is concentrated, its arteriosclerosis-preventing capability is enhanced even further, and factors that is induce symptoms of arteriosclerosis can be reduced with further reliability.

When further explained, the above-described arteriosclerosis-preventing capability is realized as a result of one or several effects, including the antioxidant activity, LDL-C-reducing activity, endothelium-derived relaxing factor (EDRF)-induced vasorelaxing activity and platelet aggregation-inhibiting activity, the ability to decomposes and eliminate cholesterol ester from foam cells that have accumulated on the surface inside vessels via macrophage uptake of oxidized LDL-C, IL-1 and IL-2-induced vasorelaxing activity, and the cholesterol-reducing effects of lactic acid bacteria, of the product of the present invention that contains large quantities of isoflavone aglycone, as previously explained.

Moreover, the immune-activating material of the present invention is characterized in that it is a product having immune-activating capability that is produced by inoculating koji mold on a pulse crop to effect koji preparation and by adding water to the resulting product of the koji preparation, thus hydrolyzing the proteins and/or saccharides contained in such a resulting product; and further by promoting a propagation of lactic acid bacteria contained in resulting product of the koji preparation and/or the lactic acid bacteria that have been added to the resulting product of the koji preparation. This immune-activating material has immune-activating capability; therefore, when it is absorbed *in vivo* from the digestive tract of a vertebrate, immunity of the vertebrate is improved, and maintenance and promotion of good health and prevention of disease, etc. is well accomplished by way of realizing its immune-activating capability. In particular, excellent immune-activating capability is realized from the immune-activating capability of both the high isoflavone aglycone and lactic acid bacteria which are contained great amount.

Furthermore, this immune-activating material is a product that also possesses arteriosclerosis-preventing activity. Accordingly, synergism is realized in terms of the immune-activating capability and arteriosclerosis-preventing capability of the present invention.

Moreover, the above-described lactic acid bacteria should be the genus *Enterococcus,* particularly at least one species selected from *Enterococcus faesium* and *Enterococcus faecalis.* By using this type of lactic acid bacteria, it is possible to realize better immune-activating capability.

In addition, the vertebrate other than humans of the present invention is characterized in that it is reared eating the above-described arteriosclerosis-preventing material or the above-described immune-activating material. In concrete terms, the vertebrate other than humans of the present invention is reared by consuming feed to which the above-described arteriosclerosis-preventing material and immune-activating material have been added. When these arteriosclerosis-preventing material and immune-activating material are absorbed *in vivo* from the digestive tract of the vertebrate, the development of arteriosclerosis in the vertebrate can be reliably prevented by the realization of arteriosclerosis-preventing capability, and maintenance and promotion of good health and prevention of disease, etc. are well accomplished; and immunity of the vertebrate can be improved by realization of immune-activating capability, and maintenance and promotion of good health and prevention of disease, etc. are also well accomplished. Furthermore, when vertebrates that have been reared in this way are used for food, the vertebrate that has been reared under very healthy conditions are used as edible meat, etc.

In addition, the egg of a vertebrate other than human beings of the present invention is characterized in that it is the egg of a vertebrate that has eaten the above-described arteriosclerosis-preventing material or the above-described immune-activating material. The components of eggs produced by fowl and fish that have ingested feed, to which the arteriosclerosis-preventing material or immune-activating material of the present invention has been added, are lower in cholesterol and have a high isoflavone aglycone content when compared to the components of eggs of chickens, etc. that have ingested conventional feed that does not Contain these materials. This is because the chickens and fish are healthy because they eat the arteriosclerosis-preventing material or immune-activating material of the present invention; and as a result, their eggs are made from healthier components.

### Brief Description of Drawings

Figure 1 is a process diagram showing one embodiment of the production method for making an arteriosclerosis-preventing material or immune-activating material from the soybean meal, which is one type of pulse crop, by the present invention;
Figure 2 is a graph showing changes in the weight of rats when arteriosclerosis-preventing capability of the arteriosclerosis-preventing material was studied;
Figure 3 is a graph showing changes in the amount of feed consumed by rats when arteriosclerosis-preventing capability of the arteriosclerosis-preventing material was studied;
Figure 4 is a graph showing changes in the TL, LDL-C, HDL-C and neutral fats (referred to hereafter ask TG) in the blood of rats when the arteriosclerosis-preventing capability of the arteriosclerosis-preventing material was studied;
Figure 5 is a graph showing the correlation between the smooth muscle cell propagation capability of rats and the amount of concentrated material, etc. added when studies were performed on the arteriosclerosis-preventing capability of the arteriosclerosis-preventing material; and
Figure 6 is another graph showing the correlation between smooth muscle cell propagation capability of rats and the amount of concentrated material, etc. added when the arteriosclerosis-preventing capability of the arteriosclerosis-preventing material was studied.

### Best Mode for Carrying Out the Preferred Embodiments and the Invention

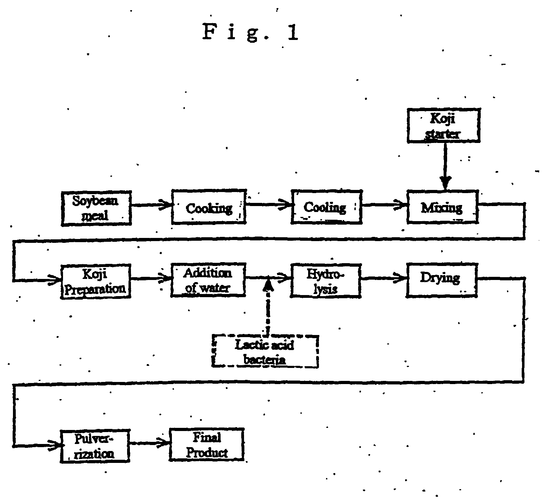

Embodiments of the present invention will be described below with reference to Figure 1 through Figure 6.

The solid line in Figure 1 shows one embodiment of the production method for making the arteriosclerosis-preventing material and immune-activating material from the soybean meal, which is one type of pulse crop, by the present invention. The chain line in the same figure shows yet another embodiment of a production method of the present invention.

First, how the arteriosclerosis-preventing material and immune-activating material of the present invention are produced in accordance with the process shown by the solid line in Figure 1 will be described.

First, the soybean meal is cooked. Propagation of the soybean meal is facilitated by this cooking when compared to when cooking is not performed. Depending on the purpose of production, etc. cooking of the soybean meal is performed by the batch method, whereby cooking and subsequent koji preparation are separately performed, or by the continuous method using a koji preparation device with which cooking and subsequent koji preparation are continuously performed.

Once cooking has been completed, the water content of the soybean meal is adjusted to the content with which propagation of koji mold can be accomplished (for instance, approximately 36 wt%).

The arteriosclerosis-preventing material and immune-activating material of the present invention are produced as follows using soybean meal whose water content is adjusted as described above.

More specifically, when the cooked soybean meal simple substance is cooled to bring its temperature to 40°C or lower so as not to kill the koji mold, the soybean meal is inoculated with koji starter consisting of koji mold to a specific weight ratio, and then these two are mixed until a uniform mixture is obtained.

So as to raise the temperature of the mixture as a result of propagation of the koji starter, the temperature of the mixture is once reduced inside a koji preparation device and the mixture is set aside while keeping temperature at approximately 32°C. As a result, the soybean meal is fermented by the koji mold, and temperature rises to approximately 38°C as koji preparation thus proceeds; and the product becomes firm and solidifies as hyphae of the propagating koji mold grow. Then, as koji preparation proceeds, the solidified product is released from the koji preparation device by being spun and agitated. Ventilation is provided in order to uniformly feed air to the inside and control temperature so that product temperature is reduced to approximately 33 - 35°C. Koji preparation proceeds further as ventilation is continued. As a result, death of the koji mold is reliably prevented, and propagation of koji mold is satisfactorily accomplished. Then, the product inside the koji preparation device is spun and agitated enough times depending on how firmly the product has solidified inside the koji preparation device as ventilation is continued.

The koji mold used for the above koji preparation is the koji mold that has been used for many years in fermented food products unique to Japan and "tempe." The genus *Aspergillus,* including *Aspergillus usamii*, *Aspergillus kawachii*, *Aspergillus awamori*, *Aspergillus saitoi*, *Aspergillus oryzae*, *Aspergillus niger*, etc. and those classified as the genus *Rhizops,* which are safe for food products, are preferably used.

The fermentation time is at least 24 hours or longer, depending on the species of koji mold that is used. The fermentation time should be long enough to thoroughly propagate the koji and decompose a specific amount of proteins and/or saccharides in the soybean meal.

In this embodiment, hydrolysis of the proteins and/or saccharides is further performed on the product of koji preparation. Here, the term saccharides includes saccharides such as sucrose, etc. and starches etc. Hydrolysis after koji preparation is performed with the substrate in flake form.

More specifically, in this embodiment, after completion of koji preparation, water is added to the product to a water content of, for instance, 50 wt%, then the product is heated to 30 - 55°C and kept at this temperature for a specific amount of time in order to hydrolyze the proteins and/or saccharides contained in the product of koji preparation. As a result of hydrolysis of the proteins and/or saccharides in this way, large amounts of isoflavone aglycone, which has arteriosclerosis-preventing capability and/or immune-activating capability, are produced.

Moreover, the product of the present invention can be obtained by the above-described hydrolysis in this embodiment as long as koji preparation is continued long enough to produce 10⁸ cfu/g or more of lactic acid bacteria. In this embodiment, the lactic acid bacteria are lactic acid bacteria that are naturally contained in the product of koji preparation. Thus, probiotics is used in this embodiment, and the lactic acid bacteria and koji mold are actively propagated flourishing together on the same substrate, which consists of the product made from soybean starting material.

In this embodiment, lactic acid bacteria were isolated from the fermented soybean meal that was produced in accordance with the production method of the present invention using *Aspergillus oryzae,* which is the koji mold for miso, as the koji mold; and the lactic acid bacteria were identified by performing the tests under the test items shown in Table 1.

**Table 1**

| Properties of Isolated Bacteria a | |
|---|---|
| Test item | Test results |
| Morphology | Coccus *1 |
| Gram staining | + *1 |
| Spores | - *1 |
| Motility | - *1 |
| Behavior in oxygen | Facultative anaerobic *1 |
| Catalase | - *1 |
| Lactic acid product | L(+) *1 |
| Production of gas from glucose | - *1 |
| Viable at 10°C | + |
| Viable at 45°C | + |
| Viable at 50°C | + |
| Viable in the presence of 6.5% NaCl | + |
| Viable at pH of 9.6 | + |
| Viable in the presence of 40% bile | + |
| Production of yellow pigment | - |
| Hemolytic (ovine blood) | α hemolysis |
| Arginine dihydrolase | + |
| Hydrolysis of equine uric acid | + |
| Hydrolysis of esculin | + |
| Viable in the presence of 0.1% methylene blue milk | + |

| Production of acid | |
|---|---|
| D-xylose | - |
| L-ramnose | + *2, *3 |
| Sucrose | + |
| Lactose | + |
| Melibiose | + |
| Raffinose | + *2 |
| Melezitose | - |
| Glycerol | - *2, *3 |
| Adonitol | - |
| Sorbitol | + *2 |
| Mannitol | + |
| L-arabinose | + |
| GC content of DNA in cell walls of bacterium (mol%) *4 | 40 |

| | |
|---|---|
| *1 Shows the results of a previous reports (Test Report No. 598010212-001) | |
| *2 Atypical property | |
| *3 Enterococcus faesium JCM5804^{T} (reference strain), however, showed the same reaction as isolated bacterium a. | |
| *4 By HPLC method. | |

Based on the properties shown in Table 1, the isolated lactic acid bacteria was identified to be *Enterococcus faesium* of the genus *Enterococcus*. This lactic acid bacteria was confirmed to be an intestinal flora calle*d Enterococcus*.

The above-described fermentation time and hydrolysis time as well as hydrolysis temperature in this embodiment should be adjusted in accordance with: the type, state, properties and quantity of pulse crop; type, state, properties and quantity of koji mold; type, state, properties and quantity of lactic acid bacteria and type, properties, etc. of the product arteriosclerosis-preventing material or immune-activating material.

The arteriosclerosis-preventing material or immune-activating material of this embodiment that is produced as described above is a product that has a high isoflavone aglycone content as the result of thorough decomposition of the proteins and/or saccharides in the soybean meal by koji preparation and subsequent hydrolysis treatment. This isoflavone aglycone has arteriosclerosis-preventing capability or immune-activating capability, and it is easily absorbed into the bloodstream from the walls of the intestine of vertebrates, thus providing early realization of arteriosclerosis-preventing capability or immune-activating capability.

More specifically, the arteriosclerosis-preventing capability, on the one hand, is realized as a result of one or several effects of the product of the present invention that contains, as described above, large quantities of isoflavone aglycone; and such effects include the antioxidant activity, LDL-C-reducing activity, endothelium-derived relaxing factor (EDRF)-induced vasorelaxing and platelet aggregation-inhibiting activities, the ability to decomposes and eliminate cholesterol ester from foam cells that have accumulated on the surface inside vessels via macrophage uptake of oxidized LDL-C, IL-1 and IL-2-induced vasorelaxing activity, and the cholesterol-reducing effects of lactic acid bacteria.

On the other hand, as to the immune-activating capability, the lactic acid bacteria, particularly *Enterococcus faesium,* which belongs to the *genus Enterococcus* that propagates to very high numbers in the substrate of the product of the present invention, possesses excellent immune-activating capability. Accordingly, when an immune-activating agent produced from the mold is absorbed *in vivo* from the digestive tract of vertebrates such as humans, livestock, fish, etc., immunity of the animal is improved and maintenance and promotion of good health and prevention of disease, etc. are well accomplished as a result of the realization of immune-activating capability.

The cholesterol-reducing capability of the present invention is considered to be as follows:
A reduction in cholesterol levels can be expected due to the synergism between the effect of the lactic acid bacteria itself that has propagated to large quantities as described above and the activity of product that is not decomposed by protease which is an enzyme that is produced by production of koji mold.

Furthermore, there is synergism between the above-described arteriosclerosis-preventing capability, immune-activating capability and cholesterol-reducing capability; and as a result, maintenance and promotion of good health and prevention of disease, etc. are accomplished.

Moreover, the necessary amount of arteriosclerosis-preventing material and immune-activating material of the present invention is added to feed for livestock, fowl, fish, etc. during rearing of such vertebrates. When the vertebrates are thus reared consuming feed to which the above-described arteriosclerosis-preventing material and immune-activating material are added, these arteriosclerosis-preventing material and immune-activating material are absorbed *in vivo* from the digestive tract of the vertebrate. With arteriosclerosis-preventing activity, the onset of arteriosclerosis in the vertebrate is reliably prevented, and maintenance and promotion of good health and prevention of disease, etc. are well accomplished. In addition, with immune-activating capability, immunity of the vertebrate is improved, and maintenance and promotion of good health and prevention of disease, etc. are accomplished well. Furthermore, when a vertebrate that has been reared in this way is used for food, a vertebrate that has been reared under very healthy conditions are used for edible meat, etc.

Furthermore, the components of eggs produced by fowl and fish that have consumed the arteriosclerosis-preventing material and immune-activating material of this embodiment have a lower cholesterol content and a higher isoflavone aglycone content compared to the components of the eggs of fowl, etc. that have consumed conventional food to which these materials are added. This is because the fowl and fish are healthy because of the arteriosclerosis-preventing material and immune-activating material of the present invention; and as the result, the egg that is obtained from such fowl and fish contains healthier components. Thus, an effective method of raising various vertebrates can be performed by adding and providing the arteriosclerosis-preventing material and immune-activating material of this embodiment in fodder and feed.

Next, another method of producing the arteriosclerosis-preventing material and immune-activating material of the present invention shown by the chain line in Figure 1 will be described.

In this embodiment, propagation of coexisting koji mold and lactic acid bacteria is promoted during hydrolysis of the product produced by koji preparation.

In other words, the same processes as in the above-described previous embodiment are performed until koji preparation has been completed; and then lactic acid bacteria is put in the product of koji preparation by either adding lactic acid bacteria to the product during hydrolysis or using lactic acid bacteria naturally contained in the pulse crop starting material.

This lactic acid bacteria should be the genus *Enterococcus*, particularly *Enterococcus faesium.*

When the lactic acid bacteria is introduced to the product of koji preparation in this way and hydrolysis is started, the koji mold, that is propagated during koji preparation, and lactic acid bacteria coexist in the above-described product, which are of the same ground substance, during the hydrolysis. Thus, the lactic acid bacteria receives nutrients from the product and promotes its propagation, and the koji mold and lactic acid bacteria are cultivated together in the product. In particular, vitamin Bs are produced in the product by koji preparation, and components that are easily absorbed and are of high nutritional value to the lactic acid bacteria are produced. As a result, propagation of the lactic acid bacteria using these components as a nutrient source is promoted. Furthermore, hydrolysis of various components by enzymes of the koji mold corresponds to decomposition of the soybean meal by digestive enzymes, and the digested product and undigested product are separated in the product of the embodiment. The separated product (digested product) that has been hydrolyzed by the enzyme of koji mold is directly absorbed when it is ingested by humans and livestock, but the undecomposed product becomes a useful nutrient source for lactic acid bacteria that are regularly present within the intestines without being absorbed and increases the production of short-chain fatty acids that are useful to humans and livestock.

Moreover, because the above-described product can be a culture medium in which lactic acid bacteria can actively propagate, the lactic acid bacteria that have propagated can constantly be present within the intestines of the livestock using enzyme undecomposed product contained in the product as the nutrient source. The arteriosclerosis-preventing material and immune-activating material are eventually produced from a pulse crop starting material that possesses the ability of promoting propagation of lactic acid bacteria and uses probiotics in which koji mold and useful microorganisms are cultivated in a product that serves as the same substrate.

The arteriosclerosis-preventing material and immune-activating material of this embodiment that are produced in this way have the same excellent capability as those described in the previous embodiment.

Next, the arteriosclerosis-preventing material and immune-activating material of the present invention will be explained with concrete examples.

Fermented soybean meal, which becomes the arteriosclerosis-preventing material and immune-activating material produced by the present invention using *Aspergillus oryzae* as the koji mold, thoroughly decomposes isoflavone compound as previously described. In other words, soybean meal has a very high content of daidzin and genistin, which are glycosides, in comparison to their content of daidzein and genistein, which are aglycones; however, fermented soybean meal produced by the method of the present invention contains very little glycoside in the form of daidzin and genistin due to decomposition and have a high aglycone content in the form of daidzein and genistein, which are produced with this decomposition.

More specifically, when, using soybean meals, koji preparation is performed for 48 hours while providing ventilation to adjust temperature to 35°C, water is added to bring the water content to approximately 50% and hydrolysis of the soybeans components and the koji mold components is performed with koji mold enzymes over a 24-hour period or longer at 45°C, then the isoflavone compound contents of the obtained soybean meal becomes as shown in Table 2. It is clear that a high content of daidzein and genistein, which are aglycone forms of isoflavone compounds is obtained.

As to soybean embryo, after soybean embryo was pre-cooked and water was added to bring the water content to approximately 37%, and the resulting product was cooked, sterilized, and cooled; and then, with respect to the mixture ratio of soybean embryo and koji mold, 200 g koji starter that had been adjusted to 8 x 10⁷ koji mold spores/g (adjusted with polished rice) were mixed per 400 kg soybean embryo. Furthermore, after cooling the product to 32°C when koji preparation was started, and koji preparation proceeded without ventilation until the product temperature reached 40°C; and when temperature reached 40°C, koji preparation proceeded with an increase in temperature being prevented while providing ventilation. The first agitation ("mori" process) was conducted for approximately 17 hours after starting. The mass of soybean embryo was broken apart and temperature was controlled while providing ventilation so that the product temperature after agitation would be approximately 35°C. Next, the second agitation ("naka" process) was performed for approximately 8 hours later, and the mass was broken apart. The third agitation ("shimai" process) was further performed as the previous time for approximately 16 hours while controlling product temperature to approximately 35°C with ventilation once again. Then koji preparation was completed, approximately 48 hours after starting, by controlling temperature while providing ventilation so that the product temperature would be approximately 38°C. Once koji preparation was completed, water content of the product was adjusted while agitating to bring the water content to 50% and the product was heated to a temperature of approximately 50°C. Then, the majority of the isoflavone compounds in the soybean embryo were hydrolyzed to aglycone form by the koji mold enzymes for 48 hours or longer. As shown in Table 3, the isoflavone compound of the soybean embryo produced by treatment by the present invention was obtained mainly in large quantities in the aglycone form of daidzein.

**Table 2**

| Daidzin | Daidzein | Genistin | Genistein |
|---|---|---|---|
| Not detected | 70 | 103 | 64 |
| (Units: mg/100 g) | | | |

**Table 3**

| Units: mg/100 g | | | |
|---|---|---|---|
| Component | | Present invention | Untreated |
| Glycoside | Daidzin | 70 | 840 |
| | Genistin | 40 | 170 |
| | Glycitin | 130 | 620 |
| | Total | 240 | 1630 |
| Aglycone | Daidzin | 680 | 10 |
| | Genistin | 130 | 2.4 |
| | Glycitin | 240 | 2.0 |
| | Total | 1050 | 14.4 |

Thus, according to the present invention, it is possible to make aglycones, which, of the isoflavone compounds in soybean meal, have strong pharmacologic activity, at a very high production ratio.

Next, the immune-activating capability of the immune-activating material of the present invention that has the above-described properties will be described based on the details of rearing studies in chicken and mice.

### Study 1 on Immune-Activating Capability

### Study object

The object of the study is to investigate the immune-activating capability based on the number of deaths during rearing of chicks.

### Study method

6,200 young chicks immediately after hatching were grouped into a comparison group and a study group each. The comparison group was provided conventional feed to which *Bacillus subtilus* (0.1 wt%) was added, and the study group was provided conventional feed to which the product (0.1 wt%) of propagation of lactic acid bacteria (*Enterococcus faesium*) as useful microorganisms in pulse crops of the present invention was added, and the number of deaths of chicks at one week, two weeks, three weeks, and four weeks of age was compared.

### Study results

The number of dead chicks at one week of age was 40 in the comparison group and 25 in the study group.

The number of dead chicks at two weeks of age was 60 in the comparison group and 50 in the study group.

The number of dead chicks at three weeks of age was 125 in the comparison group and 60 in the study group.

The number of dead chicks at four weeks of age was 170 in the comparison group and 75 in the study group.

The number of deaths was extremely high after three weeks in the comparison group; therefore, the antibiotic ampicillin was quickly administered, but an increase in deaths could not be prevented.

Based on these results, when the results at four weeks of age are compared, it was possible to keep the number of deaths among the chicks that had been provided the immune-activating material of the present invention in feed to 45% or less the number among those chicks given antibiotics, which today is the strongest and most popular countermeasure against *Bacillus subtilis*. This is because death was prevented by improvement of the immunity of the young chicks at a time when they axe the weakest as the result of the good immune-activating capability of the immune-activating material of the present invention.

### Study 2 on Immune-Activating Capability

### Study object

The general immune-activating capability of mice reared under stressful conditions was investigated based on changes in spleen properties (IL-5, IL-2, INF-γ, NK activity), and immune-activating capability of certain mucosa, such as that of the intestines, esophagus, anus, etc. are investigated based on changes in properties of the Peyer's patch (IL-5, IL-2, INF-γ, IgA antibody). Each of these factors representing spleen properties (IL-5, IL-2, INF-γ, NK activity) decrease under stress. On the other hand, each of the factors representing properties of the Peyer's patch (IL-S. IL-2, INF-γ, IgA antibody) also likewise decrease under stress.

### Study method

1) Four-week-old female mice were divided into a comparison group and study groups 1 and 2 of six mice each. The comparison group was provided conventional feed, study group 1 was provided conventional feed to which product (0.1 wt%) obtained, by propagation of lactic acid bacteria (*Enterococcus faesium*) as useful microorganism in pulse crops of the present invention had been added, and study group 2 was provided conventional feed to which product (1.0 wt%) obtained by propagation of lactic acid bacteria (*Enterococcus faesium*) as useful microorganism in the pulse crops of the present invention had been added. The animals were reared for 28 days under stressful conditions of high temperature of 38 + 1°C as the rearing temperature in comparison to the 26 + 1°C that is the normal rearing environment temperature. Autopsy was performed in all animals on Day 29, and changes in spleen properties and changes in Peyer's patch were determined by determination methods listed below.
2) Method of spleen treatment and method of Peyer's patch treatment
   With respect to the spleen (SPL), once the spleen had been excised from each animal and crushed with slide glass, cells were recovered. The cells were rinsed with cultivation liquid and then pulverized with a stainless steel mesh to make a cell suspension.
   On the other hand, with respect to Peyer's patch (PP), after excising Peyer's patch from the small intestines, it was pulverized with slide glass and filtered with a mesh filter. Then the product was centrifuged, and the serum was used in IgA determinations. The sediment was submitted to specific gravity centrifugation using percol. The cells remaining on the interface were recovered and rinsed. These prepared cells were brought to a concentration of 5 x 10⁸ cells/ml. They were then added, 200 µl at a time, to a 96-hole plate and cultivated at 37°C.
3) Method of determining each determination item
   - Determination of NK activity (of spleen)
      The cell suspensions that were prepared as described above were centrifuged at 1,600 rpm and 5°C for two minutes and then suspended in an RPMI 1640 culture medium to which 10% FCS had been added and NK activity was determined by the ⁵¹Cr liberation method.
   - Determination of IgA antibody (of Peyer's patch)
      A microplate reader (MTP-32) was used in the determinations. IgA antibody titers were determined by the fluorescent enzyme antibody method (fluorescent ELISA method). This fluorescent ELISA method involved first, adding and coating anti-mouse IgA antibody diluted to a concentration of 2 mg/ml with 0.1 M phosphate buffer, 50 µl at a time, in each well using a 96-hole black plate. Once this coating was completed, rinsing was performed three times with a buffer for rinsing (PBS/Tween) of 0.05% Tween 20 dissolved in PBS, blocking buffer of 1% BSA dissolved in PBS was added, 100 µl at a time, to each well, and blocking was performed for one hour at room temperature. Then serum from the above-described centrifugation was filtered with a 0.45 µm syringe filter and added to this and treated for one hour each with biotinated anti-mouse IgA antibody and β-D-galactosidase-bound streptavidin. After rinsing, 100 µl 0.1 mM 4-MU-β-D-galactoside were added and incubated for two hours at 37°C. The reaction was stopped by adding 100 µl 0.1 M glycine-NaOH, determinations were performed with a microplate reader and the results were represented in fluorescence units (FU).
   - Determination of cytokines
      With respect to IL-2
         Cells from the spleen and Peyer's patch of mice that had been passed through a nylon wool column were prepared to 5 x 10⁸ cells/ml as the responder using an RPMI-1640 culture medium (Sigma) containing 0.5% inactive normal mouse serum (NMS) of the same species and 5 x 10⁻⁵ M 2-mercaptoethanol(2ME: Wako Junyaku brand name). This was injected, 100 µl at a time, into a 96-hole, V-shaped microplate (Flow Laboratories, Inc.) and cultivated for five days under conditions of 37°C in the presence of 5% CO₂. The cultivation supernatant was used as the IL-2 sample. The sample was added to CTLL-2 cells (2.5 x 10³ cells) and the IL-2 units were calculated from ³H-TdR uptake of the same in accordance with probit analysis of Gillis et al.
      As to IL-5 and INF-γ
         Cells prepared by the above-described spleen treatment method and Peyer's patch treatment method were prepared with a RPMI-1640 culture medium to which 10% FCS had been added and injected into a 24-hole plate to obtain 2 x 10⁸ cell/ml/well. Then 100 µl 50 µg/ml ConA were added to bring the final concentration to 5 µl/ml and cultivated for 24 hours in an incubator at 37°C in the presence of 5% CO₂. After cultivation, the cell suspension in each well was filtered with a 0.45 µm syringe filter, and the filtrate was used so as to be served as the sample for determination of IL-5 and LNF-γ production capability. The cell cultivation supernatant was added, 50 ml at a time, to a 96-hole plate that had been coated with anti-mouse IL-5 and tNF-γ antibody, and each biotinated anti-mouse cytokine antibody was added as secondary antibody. After cultivation for one hour at room temperature, streptavidin β-galactosidase was added and cultivated for another one hour. 4-Methyl funberi β-galactosidase was added and reacted. After stopping the reaction, determinations were performed with MTP-32.
      Study results
         Changes in the properties of the spleen (IL-5, IL-2, INF-γ, NK activity) and changes in the properties of Peyer's patch (IL-5, IL-2, INF-γ, IgA antibody) in study groups 1 and 2 and the comparison group are shown in Tables 4 and 5. The determinations are represented by the mean ± standard deviation in each table, and significant difference is indicated by an asterisk * in the table at a difference of 5%.

**Table 4**

| Each Spleen Determination Item | | | |
|---|---|---|---|
| Determination item | Study group 1 | Study group 2 | Comparison group |
| IL-5 (ng/ml) | 0.90±0.30* | 1.0±0.4* | 0.3±0.1 |
| IL-2 (unit/ml) | 0.09±0.02* | 0.08±0.02 | 0.07±0.01 |
| INF-((pg/ml) | 123.5±12.1 | 143.6±10.5* | 102.5±8.3 |
| NK activity (%) | 20.6±4.1 | 23.6±3.9* | 18.5±4.3 |

| | | | |
|---|---|---|---|
| * Significant difference (5%) | | | |

**Table 5**

| Each Peyer's Patch Determination Item | | | |
|---|---|---|---|
| Determination item | Study group 1 | Study group 2 | Comparison group |
| IL-5 (ng/ml) | 0.8±0.4* | 0.9±0.4* | 0.2±0.1 |
| IL-2 (unit/ml) | 0.05±0.02 | 0.06±0.02* | 0.04±0.01 |
| INF-( (pg/ml) | 94.1±6.1 | 99.1±5.5* | 80.3±7.3 |
| IgA(FU) | 525.5±19.6* | 623.5±15.3* | 425.5±16.3 |

| | | | |
|---|---|---|---|
| * Significant difference (5%) | | | |

It is clear from Tables 4 and 5 that the good immune-activating capability possessed by the immune-activating material of the present invention acts to improve general immune-activating capability and immune-activating capability of specific mucous tissues.

In other words, the mice were under stress as a result of being reared for 28 days under a temperature that was 10°C higher than normal rearing conditions for mice. In contrast to the reduction in each factor showing the properties of the spleen and Peyer's patch in the comparison group, each factor showing the properties of the spleen and Peyer's patch was significantly higher than those of the comparison group in study groups 1 and 2 wherein the immune-activating material of the present invention had been added to feed. Looking, in Table 4, particularly at the NK activity which demonstrates well the properties of the spleen, it is clear that there is marked improvement in study group 1 at 23.6 ± 3.9 when compared to the 18.54 ±4.3 of the comparison group. Therefore, it is clear that general immune-activating capability has been markedly improved by the material of the present invention. Moreover, looking, in Table 5, particularly at IgA antibody, which demonstrates well the properties of Peyer's patch, there is marked improvement in study group 1 at 525.5 ±5, 19.6 and study group 2 at 623.5 ± 15.3 when compared to the comparison group of 425.5 ± 16.3. Therefore, it is confirmed that antigen that increases IgA antibody of the intestinal mucosa immune system is present in the material of the present invention. The immune system can be regarded as the general immune system and local immune systems, and the local intestinal mucosa immune system has received considerable attention in recent years as an immune system for oral infection, It has been reported that when oral per-mucosa immune tolerance of this immune system is established with antigen, excretion of IgE antibody by the general immune system is inhibited. IgE antibody is said to be antibody related to the onset of food allergies and hay fever, etc., and it may be possible to prevent food allergies and hay fever, which have recently become problems, by activation of intestinal mucosa immunity. Therefore, there is a demand for a nontoxic, safe antigen that will activate the intestinal mucosa immune system. It is confirmed that the material of the present invention contains safe antigen to at least the intestinal mucosa immune system. Further, there is also a report that production of TGF-β is related to activation of IgA antibody (Sonoda, E., Matsumoto, R. et al.: Exp. Med., 170:1415-1420, 1989), it is possible to indicate that activation of intestinal immunity may be related to prevention of arteriosclerosis. In addition, the fact that activation of not only the intestinal mucosa immune system but also the general immune system by way of the material of the present invention increases NK activity indicates that results in terms of cancer prevention, etc. is expected.

In the meantime, there are no conventional food products that has the ability to improve IgA antibody as shown in Table 5.

The arteriosclerosis-preventing capability of the arteriosclerosis-preventing material of the present invention that has the above-described properties will now be described based on the details of rearing studies in rats.

### Study 1 Pertaining to Arteriosclerosis-Preventing Capability

### Study object

Rats on which oopharectomy had been performed were reared on food to which casein had been added (refer to Table 6) so that rats become susceptible to arteriosclerosis. These rats were orally administered for five consecutive weeks an arteriosclerosis-preventing material (referred to hereafter as concentrated material) obtained by further concentration of the arteriosclerosis-preventing material of the present invention. Changes in body weight and changes in food consumption of the rats during this time, and arteriosclerosis-preventing capability based on TC, LDL-C, HDL-C and TG VALUES of the blood when oral administration is completed were investigated.

Rats on which oopharectomy had been performed were used as the samples by the following reason: arteriosclerosis suddenly progresses in post-menopausal female vertebrates due to a sudden reduction in secretion of estrogen, and therefore, there is a marked increase in body weight, food consumption, TC, LDL-C, HDL-C and TG VALUES; accordingly, arteriosclerosis-preventing capability can be evaluated base on determinations of these changes.

Daidzein accounts for approximately 70% of the pulse crop product of the present invention which is the product of conversion of the isoflavone compound in soybean embryo to its aglycone form by the enzymes of koji mold. Because daidzein has strong estrogen-like activity when compared to genistein, lipometabolism anomaly-improving effects is expected in rats on which oopharectomy had been performed. The concentrated extract of isoflavone aglycone used in the study was prepared by defatting with hexane and then extraction and concentration with a solvent (for instance, ethanol) to approximately 30 wt% (23.4 wt% daidzein, 8.0 wt% glycitein, and 1.5 wt% genistein). Its isoflavone aglycone composition is shown in Table 7.

**Table 6**

| Feed Composition Table | | |
|---|---|---|
| | Labeled composition | |
| Feed composition | Casein | 22.7% |
| | Sucrose | 41.5% |
| | Corn starch | 20.0% |
| | Cellulose powder | 5.6% |
| | Corn oil | 5.7% |
| | Mineral mixture (AIN-76) | 3.5% |
| | Vitamin mixture (AIN-76) | 1.0% |
| | Total | 100.0% |
| Mineral mixture (in 100g) | CaHPO₄ | 50g |
| | NaCl | 7.4g |
| | K₃C₆H₅O₇·H₂O | 22g |
| | K₃SO₄ | 5.2g |
| | MgO | 2.4g |
| | MNCO₃ | 0.35g |
| | Fe.citrate(Fe17%) | 0.6g |
| | ZnCO₃ | 0.16g |
| | ZnCO₃.·Cu(OH)₂·H₂O | 0.03g |
| | Na₂SeO₃·5H₂O | 0.001 g |
| | KIO₃ | 0.001g |
| | CrK(SO₄)₂·12H₂O | 0.055g |
| | Brought to 100 g with sucrose | |
| Vitamin Mixture (in 100 g) | Vitamin A•acetate | 40000IU |
| | Vitamin D₃ | 40000IU |
| | Vitamin E·acetate | 500mg |
| | VitaminK₃ | 0.5mg |
| | Vitamin B₁ hydrochloride | 60mg |
| | Vitamin B₃ | 60mg |
| | Vitamin B₆ hydrochloride | 70mg |
| | Vitamin B₁₂ | 0.1mg |
| | D-biotin | 2mg |
| | Folic acid | 20mg |
| | Calcium pantothenate | 160mg |
| | Nicotinic acid | 300mg |
| | Choline bitartrate | 20g |
| | Brought to 100 g with sucrose | |

**Table 7**

| Composition of Concentrated Material of the Present Invention | | |
|---|---|---|
| Analysis Item | Standard Value | Analytical Value |
| Properties | Odorless, tasteless pale brown powder with no impurities | Passed |
| Weight loss by drying | 5% or less | 2.8% |
| Ignition residue | 3% or less | 0.1% |
| Arsenic | 2 ppm or less | Passed |
| Heavy metal | 20 ppm or less | Passed |
| Residual pesticide | None detected | None detected |
| General number of live bacterial | 3,000 bacteria/g or less | 3,000 bacteria/g or less |
| *Escherichia coli* group | Negative | Negative |
| Isoflavone aglycone content | 30% or more | 32.9% |
| Daizin | | 23.4% |
| Glycitein | | 8.0% |
| Genistein | | 1.5% |

### Study method

1) In order to evaluate differences based on whether or not oopharectomy had been performed, rats on which oopharectomy had been performed (nine weeks old) and pseudo-surgery rats on which surgery had been performed without excising ovaries (nine weeks old) were used. The female rats were acclimated for one week or longer, and then the healthy rats were reared as samples in groups of uniform body weight.
2) A medium of Japan Pharmacopoeia carmelose sodium dissolved in Japan Pharmacopoeia water for injection to a concentration of 0.5 wt% and a suspension of a known concentration of the 2 types of the standard amount of the concentrated material and isoflavone glycoside mixed in this medium were used as the substances administered to the rats.
3) Eight pseudo-surgical rats were placed in comparison group 1, and 8 oopharectomy rats each were placed in comparison groups 2 and 3 and study groups 1 and 2, and the administration substance shown in Table 8 was orally administered to each rat of each group once a day at a ratio to most recent body weight of 10 ml/kg. The substances were prepared so that the amount of isoflavone that becomes aglycone that is administered is the dose in the same table in comparison groups 3 and study groups 1 and 2.

**Table 8**

| Administration group | | Administration substance | Dose (mg/kg) | Number of Animals |
|---|---|---|---|---|
| Comparison Group 1 | Pseudo-surgery | Medium | --- | 8 |
| Comparison Group 2 | Oopharectomy | Medium | --- | 8 |
| Comparison Group 3 | Oopharectomy | Medium + isoflavone Glycoside | 80 | 8 |
| Study group 1 | Oopharectomy | Medium + concentrated Material | 200 | 8 |
| Study group 2 | Oopharectomy | Medium + concentrated Material | 50 | 8 |

4) Determination of body weight
Body weight of all rats was determined the day administration was started and once a week thereafter.
5) Amount of feed consumption
The amount of feed consumed in two days was determined once a week in all rats and the daily average feed consumption was calculated.
6) Blood biochemistry
The TC, LDL-C, HDL-C and TG VALUES of blood taken from the abdominal aorta under ether anesthesia the day after completing five weeks of administration were determined in all rats.
7) Statistical treatment of data
Test of equality of variance by Bartlett's method were performed on the quantitative data of each group, and when there was equality of variance, variance analysis was performed by the one-dimensional layout method. If there was a significant difference, group comparison with the control group was conducted by the Dunnett method. On the other hand, if there was not equality of variance, the Kruskal-Wallis test was performed, and when there was a significant difference, group comparison with the control group was performed by the rank-sum Dunnett method. The tests were all two-way tests, and the significance level was 5 and 1%.

### Study results

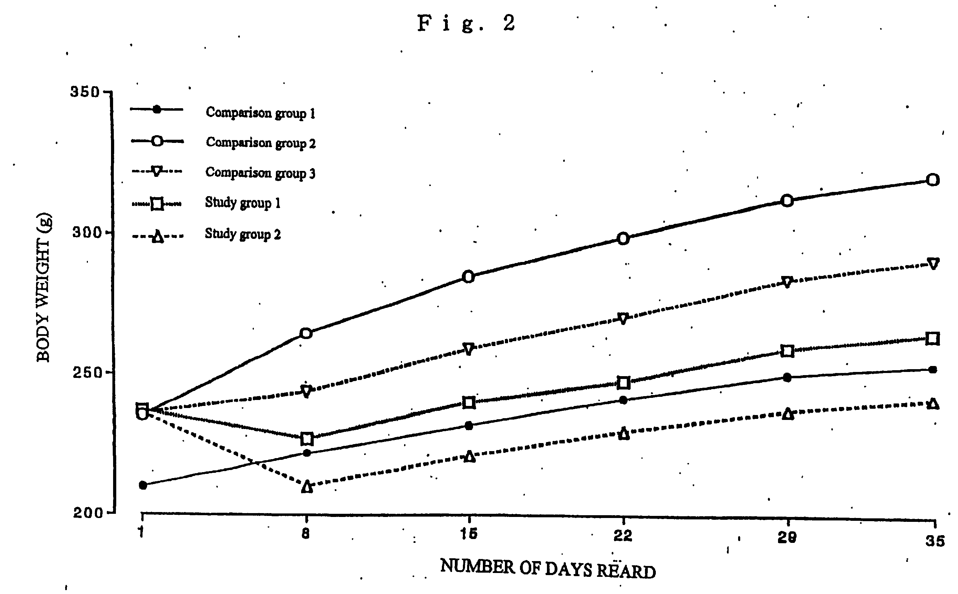

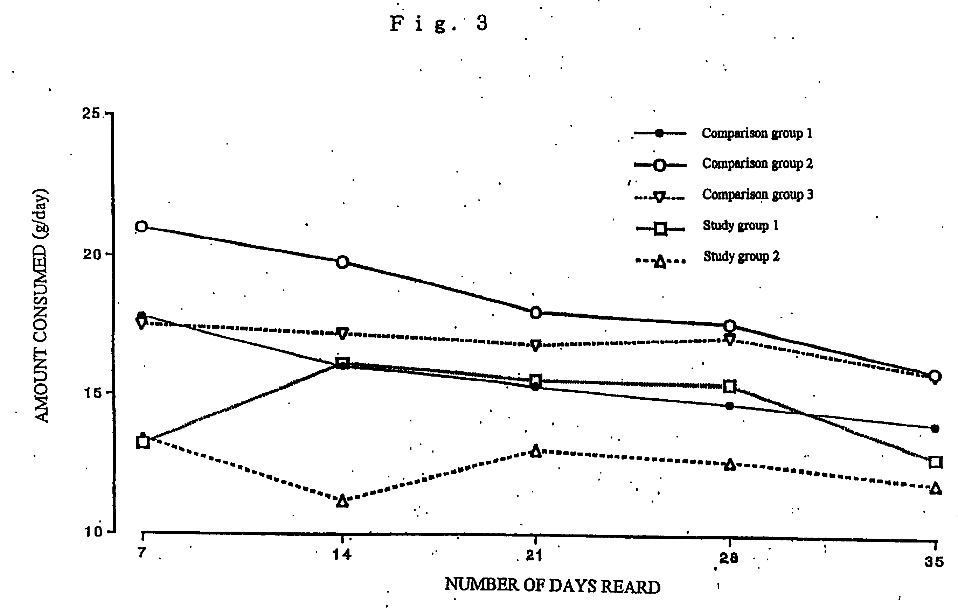

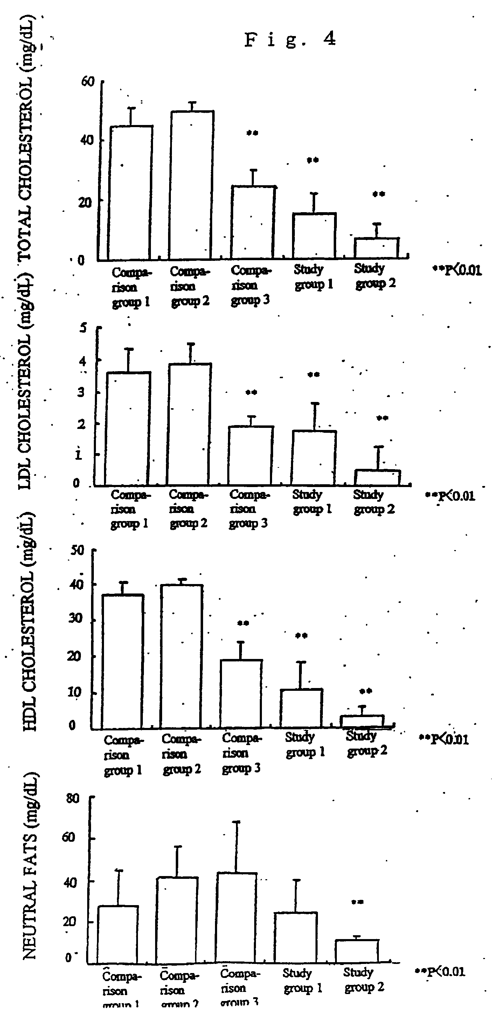

1) The results of determining body weight are shown in Figure 2.
2) The results of determining feed consumption are shown in Figure 3.
3) The results of determining TC, LDL-C, HDL-C and TG VALUES of blood are shown in Table 9 and Figure 4.

**Table 9**

| Determination item | Comparison Group 1 | Comparison Group 2 | Comparison Group 3 | Study Group 1 | Study group 2 |
|---|---|---|---|---|---|
| Total cholesterol | 44.8±6.0 | 50.1±3.1 | 24.4±5.7** | 15.0±7.0** | 6.6±5.1** |
| LDL cholesterol | 3.6±0.74 | 3.88±0.64 | 1.88±0.35** | 1.75±0.89** | 0.50±0.76** |
| HDL cholesterol | 36.9±3.6 | 39.8±1.6 | 19.0±4.9** | 10.6±7.5** | 3.1±2.7** |
| TG values | 27.6±17.1 | 41.1±15.0 | 43.4±24.4 | 24.0±15.6 | 10.8±2.1** |

| | | | | | |
|---|---|---|---|---|---|
| **P<0.01 (significant difference) | | | | | |

It is clear from the changes in body weight shown in Figure 2 that the arteriosclerosis-preventing material of the present invention has good arteriosclerosis-preventing capability and prevents an increase in rat body weight and advancement of arteriosclerosis.

More specifically, when compared to the pseudo-surgery rats of comparison group 1, body weight increased by 25g due to excision of ovaries during the rearing stage prior to administration of administration substance in the oophorectomy rats of comparison groups 2 and 3 and study groups 1 and 2. It is clear that when administration substance was administered, body weight of the rats increased, with this tendency toward an increase remaining unchanged, because there was insufficient arteriosclerosis-preventing capability induced by the administration substance in comparison groups 2 and 3; however, there was sufficient realization of arteriosclerosis-preventing capability of the concentrated material of the present invention, which was concentrated administration substance in the aglycone form, in study groups 1 and 2, and there was a reduction in body weight the first one week and a tendency toward the same change in body weight as the pseudo-surgical rats in comparison group 1, thus effectively preventing an increase in body weight.

It is clear from the changes in feed consumption shown in Figure 3 that the arteriosclerosis-preventing material of the present invention has good arteriosclerosis-preventing capability so that an increase in feed consumption by the rats was kept low and advancement of arteriosclerosis was inhibited.

More specifically, it is clear that though feed consumption by rats was high in comparison groups 1 through 3; feed consumption of rats in study groups 1 and 2 administered concentrated material of the present invention, which was concentrated aglycone form, was kept low, and advancement of arteriosclerosis was inhibited.

It is clear from the changes in TC, LDL-C, HDL-C and TG values in food consumption blood shown in Table 9 and Figure 4 that the arteriosclerosis-preventing material of the present invention has good arteriosclerosis-preventing capability; as a result, each of such values of the rats is kept low, and advancement of arteriosclerosis is prevented.

In other words, there is an increase in each of TC, LDL-C, HDL-C and TG values of the oopharectomy rats of comparison group 2 when compared to the pseudo-surgical rats in comparison group 1 due to a sudden decrease in secretion of estrogen. Also, each of TC, LDL-C, and HDL-C values of rats administered isoflavone glycoside in comparison group 3 were reduced by only approximately 1/2 as much as in comparison group 1, and TG when compared to comparison group 1. Therefore, effective arteriosclerosis-preventing capability is not realized.

In contrast to this, there was a large reduction in all of TC, LDL-C, HDL-C and TG values of rats administered concentrated material of the present invention, which was concentrated aglycone form, of study groups 1 and 2. Judging from LDL-C, which is the most important factor in the onset of arteriosclerosis, the aglycone content drops to 1/2 or less that of comparison group 1 in study group 1, which had a low aglycone content, while that of study group 2 with a high aglycone content dropped to 1/5 or less that of comparison group 1. Thus, it is clear that effective arteriosclerosis-preventing activity is fully realized. Moreover, each of TC and HDL-C were reduced to 1/3 or less that of comparison group 1 in study group 1 with a low aglycone content, while they were reduced to 1/7 and 1/11 or less that of comparison group 1 in study group 2 with a high aglycone content. Furthermore, TG was reduced to approximately 2/3 or less that of comparison group 1 in study group 2 with a high aglycone content and was also lower than that of comparison group 1 in study group 1 with a low aglycone content Consequently, it is clear from these results that the arteriosclerosis-preventing material of the present invention has good arteriosclerosis-preventing capability, keeping each such values in rats low and inhibiting advancement of arteriosclerosis.

Oopharectomy rats are menopausal female model animals. In other words, the risk of arteriosclerosis increases in post-menopausal women due to secretion of hormones, leading to lipometabolism anomalies and susceptibility to hyperlipidemia. However, according to the results of the present study, improvement of lipometabolism in menopausal females is expected with the material of the present invention (isoflavone aglycone concentrate). Although soybean proteins have shown the ability to inhibit ingestion of cholesterol in the diet, it was necessary in this case to ingest the soybean proteins with food. Nevertheless, the product of the present invention can be consumed as a supplement without replacing proteins in the diet with soybean protein. Thus, blood cholesterol is controlled without any stress on the diet.

### Study 2 on Arteriosclerosis Capability

### Study object

The object of the study is to investigate the effect of the above-described concentrated material of the present invention in terms of preventing propagation of smooth muscle cells of the tunica media of the blood vessels, which is one factor in arteriosclerosis.

### Study method

1) Smooth muscle cells (SMC) derived from the aortic tunica media of stroke-prone spontaneously hypertensive rats (SHRSP) were prepared by the explant method, and five generations were cultivated.
2) After inoculating 10,000 well SMC in a 24-well plate, the cells were rinsed in the subconfluent state with DMEM medium containing 10% FBS at 37°C and in the presence of 5% CO₂. [The medium] was replaced with DMEM medium containing 0.4% albumin (phenol red-free) and cultivated for 48 hours.
3) Genistein (referred to hereafter as Gen), daidzein (referred to hereafter as Dai), glycitein (referred to hereafter as Gly), and the concentrated material of the present invention were each added to DMEM culture medium (phenol red-free) containing 10% FBS (charcoal-treated) to a concentration of 0.1 to 30 µmol/liter and cultivated for five days. Then, the number of cells was determined with a Coulter counter (referred to hereafter as Determination A).
4) The number of cells was determined by adding 20 ng/ml PDGF in accordance with the method 3) described above (referred to as Determination B).

### Study results

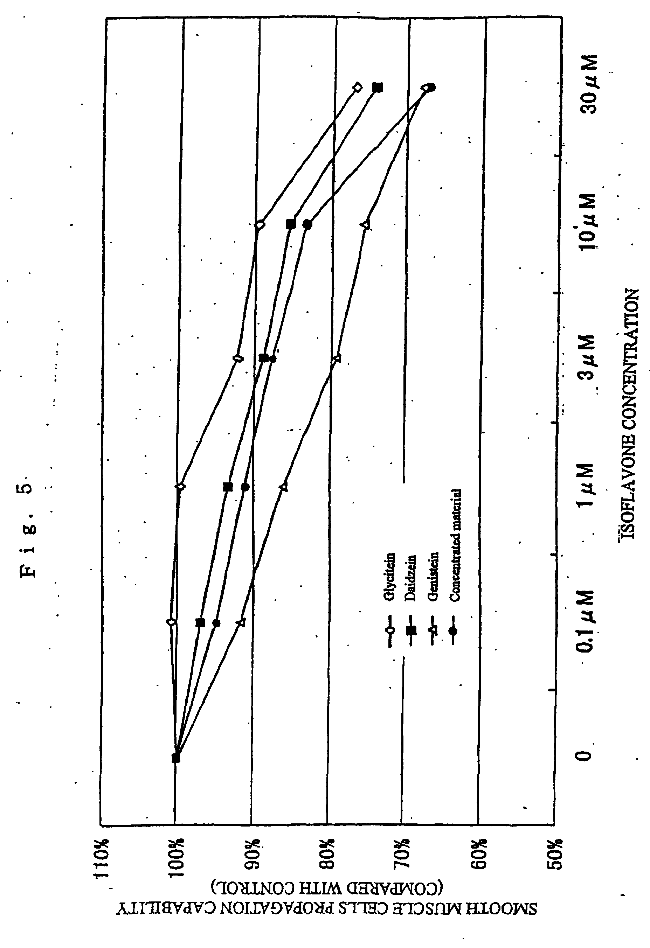

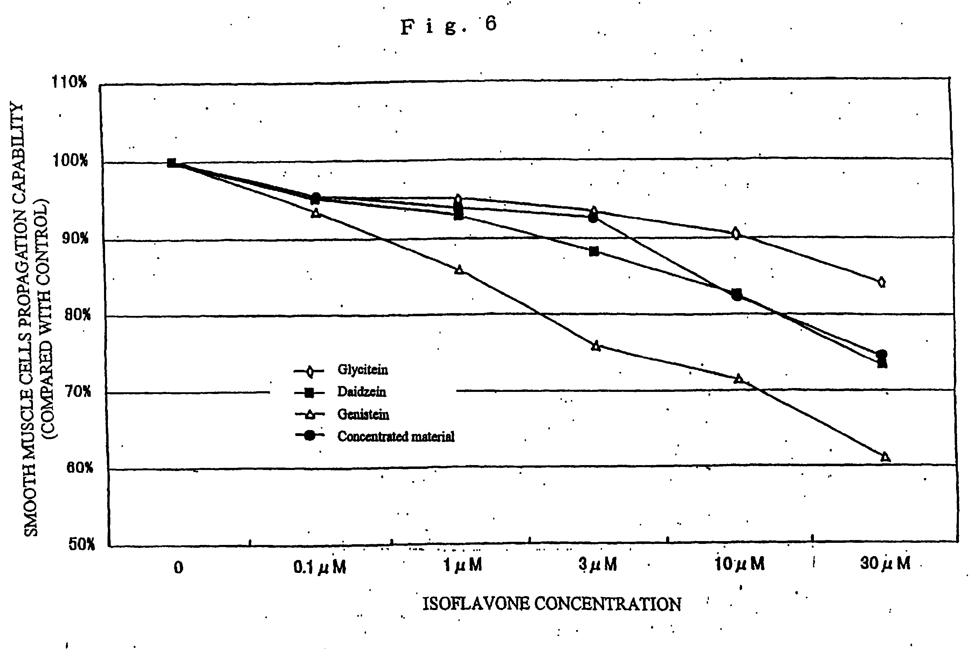

1) The results of Determination A are shown in Figure 5.
2) The results of Determination B are shown in Figure 6.

As shown in Figures 5 and 6, there is a reduction in smooth muscle cell propagating capability (when compared to the control) with an increase in the isoflavone concentration. In particular, it is clear that almost the same cell-reducing effect as with Gen only and Dai only is obtained with the concentrated material of the present invention. Consequently, the concentrated material of the present invention has the effect of inhibiting propagation of tunic media smooth muscle cells of the blood vessels, which is one factor in arteriosclerosis; therefore, excellent arteriosclerosis-preventing capability is realized.

In arteriosclerosis, lipofilaments form in the blood vessels upon uptake of oxidized LCL-C by macrophages; and during the final stages of arteriosclerosis, meandering and propagation of smooth muscle cells of the tunica media of blood vessels occur, and clogging of vessels tends to occur. However, the effect of inhibiting propagation of smooth muscle cells with relatively low concentration was seen with isoflavone aglycone concentrated the product of the present invention in the study described above. This indicates that stopping further advancement in people in whom arteriosclerosis has already progressed is expected; and thus, the product of the present invention is very effective material in preventing arteriosclerosis.

Moreover, when soybean meal produced as described above is used as feed, etc., it can be used as pulverized soybean meal by drying soybean meal that has been obtained as in each of the above-described embodiments and then pulverized. In addition, in the case of the immune-activating material that uses the cell walls of the lactic acid bacterium, the product can be heated and sterilized, because activity is realized even with dead bacterium.

Moreover, a conventional koji preparation device can be used as is in the present invention. It is not particularly necessary to build devices with an industrial base. Thus, the present invention is high in general-purpose usage.

Furthermore, the present invention is not limited to each of the above-described embodiments, and modifications can be made as needed.

## Claims

1. An arteriosclerosis-preventing material characterized in that said material is produced by inoculating koji mold on a pulse crop to effect koji preparation and by adding water to a product of said koji preparation, thus hydrolyzing proteins and/or saccharides contained in said product of said koji preparation, so that said material possesses arteriosclerosis-preventing capability.

2. An arteriosclerosis-preventing material according to Claim 1, characterized in that propagation of lactic acid bacteria contained in said product of said koji preparation and/or lactic acid bacteria that has been added to said product of said koji preparation is promoted during said hydrolysis.

3. An arteriosclerosis-preventing material according to Claim 2, characterized in that said lactic acid bacteria belongs to genus *Enterococcus.*

4. An arteriosclerosis-preventing material according to Claim 3, characterized in that said genus *Enterococcus* is at least one species of *Enterococcus faesium* and *Enterococcus faecalis.*

5. An arteriosclerosis-preventing material according to any one of Claims 1 through 4, characterized in that said product that has arteriosclerosis-preventing capability is in a concentrated state.

6. An immune-activating material characterized in that said material is produced by inoculating koji mold on a pulse crop to effect koji preparation and by adding water to a product of said koji preparation, thus hydrolyzing proteins and/or saccharides contained in said product of said koji preparationm, and further, upon said hydrolysis, by promoting propagation of lactic acid bacteria contained in said product of said koji preparation and/or lactic acid bacteria added to said product of said koji preparation, so that said material possesses immune-activating capability.

7. An immune-activating material according to Claim 6, characterized in that said immune-activating material is a product that also has arteriosclerosis-preventing capability.

8. An immune-activating material according to Claim 6 or Claim 7, characterized in that said lactic acid bacteria belongs to genus *Enterococcus*.

9. An immune-activating material according to Claim 8, characterized in that said genus *Enterococcus* is at least one species of *Enterococcus faesium* and *Enterococcus faecalis.*

10. A vertebrate other than a human being that has eaten said artetiosclerosis-preventing material of any one of Claims 1 through 5 or said immune-activating material of anyone of Claims 6 through 9.

11. An egg of a vertebrate other than a human being that has eaten said arteriosclerosis-preventing material of any one of Claims 1 through 5 or said immune-activating material of any one of Claims 6 through 9.
